# EUROPEAN PATENT SPECIFICATION

(11) **EP 4 119 176 B1**
(45) Date of publication and mention of the grant of the patent: **05.03.2025**
(21) Application number: 20923769.2
(22) Date of filing: 11.03.2020
(51) Int. Cl.: A61M 16/00, A61M 16/20

(54) **RESPIRATORY VENTILATION SYSTEM AND METHOD**
BEATMUNGSSYSTEM UND VERFAHREN
SYSTÈME ET PROCÉDÉ DE VENTILATION RESPIRATOIRE

(43) Date of publication of application: 18.01.2023
(73) Proprietor: Shenzhen Mindray Bio-Medical Electronics Co., Ltd., Shenzhen, Guangdong 518057 (CN)
(72) Inventor: CAI, Kun, Shenzhen, Guangdong 518057 (CN); WU, Leping, Shenzhen, Guangdong 518057 (CN); LIU, Huawang, Shenzhen, Guangdong 518057 (CN); XIAO, Yang, Shenzhen, Guangdong 518057 (CN)
(74) Representative: KIPA AB
(86) International application number: PCT/CN2020/078844
(87) International publication number: WO 2021/179215

(56) References cited:
- WO-A1-2014/046583
- WO-A1-2014/111832
- WO-A2-2017/144963
- CN-A- 1 074 620
- CN-A- 101 622 036
- CN-A- 102 274 565
- CN-A- 103 889 491
- CN-A- 104 640 590
- CN-A- 105 579 089
- US-A1- 2008 245 366
- US-A1- 2014 283 834

## Description

### TECHNICAL FIELD

The disclosure relates to the technical field of medical devices, and more particularly to a respiratory ventilation system.

### BACKGROUND

Respiratory disturbance and failure is one of the main reasons that affect the survival rate of newborns, especially premature infants. In some cases, the traditional ventilation method for newborns cannot effectively carry out respiratory support. This is because the airway pressure provided by the traditional ventilation method is high, which may lead to pulmonary injury. In view of this situation, the high-frequency oscillation ventilation method can be used for respiratory support.

High-frequency oscillation ventilation has three basic characteristics. Its respiratory rate reaches 3-50HZ, its tidal volume approaches to physiological dead space of the patient, and it has active inhalation and exhalation. Among them, in contrast to the traditional ventilation mode that only ventilates during the inspiratory phase and depends on human physiological response ventilation during the expiratory phase, high-frequency oscillation ventilation can realize active exhalation, that is, during the expiratory phase of the patient, the machine generates negative force to help the patient to exhale, which is extremely critical for newborns or other patients with pulmonary diseases. In addition, ventilation parameters of high-frequency oscillation ventilation include oxygen concentration, mean pressure, frequency, amplitude and respiratory ratio, which can be preset and regulated according to own state and treatment need of the patient.

At present, the high-frequency oscillation ventilation device that realizes the high-frequency oscillation ventilation method, for example, the high-frequency oscillation ventilator, has two implementation ways. One of which is to use the oscillation method which is similar to the loudspeaker diaphragm to control the active inhalation and active exhalation by forward and backward movements of a diaphragm, which is driven by a motor to control the movement stroke and frequency. However, the larger the required oscillation amplitude, the larger the device volume. Moreover, it is difficult to combine traditional ventilation and high-frequency ventilation. The other of which is to use solenoid valve to realize active inhalation and venturi negative pressure suction device to realize active exhalation. However, venturi negative pressure needs high-pressure gas drive, which is noisy and consumes too much gas source.

D1 (WO2014/046583) teaches a breathing apparatus provides high frequency oscillatory ventilation to a patient by supplying breathing gas to the patient according to an oscillating pressure profile oscillating between a positive pressure and a negative pressure. However, such breathing apparatus uses the same inspiration valve for active inhalation and active exhalation, which is not effective, consumes a large volume of gas and generates a lot of noise.

### SUMMARY

In order to solve the above technical problems, embodiments of this disclosure expects to provide a respiratory ventilation system and method, which uses active expiratory device to extract exhaled gas of patient to produce active exhalation, so as to support a high-frequency ventilation mode, which not only has a small system volume, but also reduces gas consumption and noise.

The technical schemes of embodiments of this disclosure can be realized as follows.

According to an embodiment of this disclosure, a respiratory ventilation system is provided, which including a gas source interface, an inspiratory branch, a high-frequency oscillation generation device, a ventilation control device, and an active expiratory device;
the inspiratory branch is respectively connected with the gas source interface and a patient pipeline which is connected with a respiratory system of a patient;
the high-frequency oscillation generation device is configured to generate high-frequency oscillation for a gas of the inspiratory branch;
the ventilation control device is connected with the inspiratory branch, the high-frequency oscillation generation device and the active expiratory device, wherein the ventilation control device is configured to control the high-frequency oscillation generation device to generate the high-frequency oscillation for the gas of the inspiratory branch according to a preset high-frequency oscillation frequency and to output a high-frequency oscillation gas which is generated by the high-frequency oscillation generation device through the inspiratory branch and the patient pipeline during an inspiratory phase, and is also configured to control the active expiratory device to actively extract, according to the preset high-frequency oscillation frequency, a gas which is exhaled by the patient through the patient pipeline during an expiratory phase.

In the above respiratory ventilation system, the active expiratory device includes a high-frequency valve and/or an electric gas extraction device;
the respiratory ventilation system further includes an expiratory branch which is connected with the patient pipeline to discharge the exhaled gas of the patient;
the active expiratory device is arranged at the inspiratory branch or the expiratory branch.

In the above respiratory ventilation system, the high-frequency valve is a switching valve or a proportional valve.

In the above respiratory ventilation system, the ventilation control device is further configured to control the active expiratory device to be switched off, so as to discharge the exhaled gas of the patient through the patient pipeline and the expiratory branch.

In the above respiratory ventilation system, during the expiratory phase, the ventilation control device is further configured to, control the high-frequency valve to open, and control an opening size of the high-frequency valve and extraction power of the electric gas extraction device according to the preset high-frequency oscillation frequency, so as to actively extract the exhaled gas of the patient through the patient pipeline; or
during the expiratory phase, the ventilation control device is further configured to, regulate an opening size of the high-frequency valve according to the preset high-frequency oscillation frequency, so as to actively extract the exhaled gas of the patient through the patient pipeline; or
during the expiratory phase, the ventilation control device is further configured to, regulate extraction power of the electric gas extraction device according to the preset high-frequency oscillation frequency, so as to actively extract the exhaled gas of the patient through the patient pipeline.

In the above respiratory ventilation system, the gas source interface includes a first gas source interface and a second gas source interface, the inspiratory branch includes a first gas supply branch, a second gas supply branch and a third gas supply branch;
wherein a gas outlet end of the first gas supply branch and a gas outlet end of the second gas supply branch are respectively connected with a gas inlet end of the third gas supply branch;
a gas outlet end of the third gas supply branch is connected with the patient pipeline;
a gas inlet end of the first gas supply branch is connected with the first gas source interface;
a gas inlet end of the second gas supply branch is connected with the second gas source interface.

In the above respiratory ventilation system, the first gas supply branch includes a first inspiratory check valve and a first flow regulation valve which are connected in sequence, the second gas supply branch includes a second inspiratory check valve and a second flow regulation valve which are connected in sequence, and the third gas supply branch includes a third inspiratory check valve;
the first inspiratory check valve is connected with the first gas source interface, and the second inspiratory check valve is connected with the second gas source interface.

In the above respiratory ventilation system, the high-frequency oscillation generation device and the third inspiratory check valve are connected in sequence;
the first flow regulation valve and the second flow regulation valve are respectively connected with the high-frequency oscillation generation device.

In the above respiratory ventilation system, the high-frequency oscillation generation device is composed of the first flow regulation valve and the second flow regulation valve.

In the above respiratory ventilation system, the ventilation control device is configured to control the active expiratory device to work together with the high-frequency oscillation generation device to control an average airway pressure.

In the above respiratory ventilation system, the high-frequency oscillation generation device is a high-frequency inspiratory valve.

In the above respiratory ventilation system, the high-frequency inspiratory valve is any one of proportional solenoid valve, blocking valve, servo valve and turbine.

In the above respiratory ventilation system, the active expiratory device is connected with an outlet of the third inspiratory check valve, or is connected with the expiratory branch.

According to an embodiment of this disclosure, a respiratory ventilation method (not claimed) is provided, which is applied to the above respiratory ventilation system and includes:
during the inspiratory phase, controlling the high-frequency oscillation generation device to generate the high-frequency oscillation for the gas of the inspiratory branch according to the preset high-frequency oscillation frequency, and transmitting the generated high-frequency oscillation gas to the patient through the inspiratory branch and the patient pipeline, by the ventilation control device;
during the expiratory phase, controlling the active expiratory device to actively extract the exhaled gas of the patient through the patient pipeline according to the preset high-frequency oscillation frequency, by the ventilation control device.

In the above respiratory ventilation method, the active expiratory device includes a high-frequency valve and/or an electric gas extraction device; wherein during the expiratory phase, controlling the active expiratory device to actively extract the exhaled gas of the patient through the patient pipeline according to the preset high-frequency oscillation frequency, by the ventilation control device, includes:
during the expiratory phase, controlling the high-frequency valve to open and controlling an opening size of the high-frequency valve and extraction power of the electric gas extraction device according to the preset high-frequency oscillation frequency, by the ventilation control device, so as to actively extract the exhaled gas of the patient through the patient pipeline; or
during the expiratory phase, regulating an opening size of the high-frequency valve according to the preset high-frequency oscillation frequency by the ventilation control device, so as to actively extract the exhaled gas of the patient through the patient pipeline; or
during the expiratory phase, regulating extraction power of the electric gas extraction device according to the preset high-frequency oscillation frequency, by the ventilation control device, so as to actively extract the exhaled gas of the patient through the patient pipeline.

The respiratory ventilation method further includes:
controlling the active expiratory device to work together with the high-frequency oscillation generation device by the ventilation control device, so as to control an average airway pressure.

Embodiments of this disclosure disclose a respiratory ventilation system which includes a gas source interface, an inspiratory branch, a high-frequency oscillation generation device, a ventilation control device, and an active expiratory device; wherein the inspiratory branch is respectively connected with the gas source interface and a patient pipeline which is connected with a respiratory system of a patient; the high-frequency oscillation generation device is configured to generate high-frequency oscillation for a gas of the inspiratory branch; the ventilation control device is connected with the inspiratory branch, the high-frequency oscillation generation device and the active expiratory device, and is configured to control the high-frequency oscillation generation device to generate the high-frequency oscillation for the gas of the inspiratory branch according to a preset high-frequency oscillation frequency and output a high-frequency oscillation gas which is generated by the high-frequency oscillation generation device through the inspiratory branch and the patient pipeline during an inspiratory phase, and is configured control the active expiratory device to actively extract, according to the preset high-frequency oscillation frequency, a gas which is exhaled by the patient through the patient pipeline during an expiratory phase. The respiratory ventilation system provided by embodiments of this disclosure uses the active expiratory device to extract the exhaled gas of the patient to produce active exhalation, so as to support the high-frequency ventilation mode, which not only has a smaller system volume, but also reduces gas consumption and noise.

### BRIEF DESCRIPTION OF THE DRAWINGS

FIG. 1 is a first structural schematic block diagram of a respiratory ventilation system provided by an embodiment of this disclosure.
FIG. 2 is a second structural schematic block diagram of a respiratory ventilation system provided by an embodiment of this disclosure.
FIG. 3 is a third structural schematic block diagram of a respiratory ventilation system provided by an embodiment of this disclosure.
FIG. 4 is a fourth structural schematic block diagram of a respiratory ventilation system provided by an embodiment of this disclosure.
FIG. 5 is a fifth structural schematic block diagram of a respiratory ventilation system provided by an embodiment of this disclosure.
FIG. 6 is a flow diagram of a respiratory ventilation method provided by an embodiment of this disclosure.
FIG. 7 is a detailed control diagram of an exemplary ventilation control device provided by an embodiment of this disclosure.
FIG. 8 is a control flow diagram of an exemplary ventilation control device provided by an embodiment of this disclosure.

### DESCRIPTION OF THE PREFERRED EMBODIMENTS

In order to understand the characteristics and technical content of the embodiment of this disclosure in more detail, the implementations of embodiments of this disclosure are described in detail below in combination with the attached drawings. The attached drawings are for reference and explanation only, and are not used to define the embodiments of this disclosure.

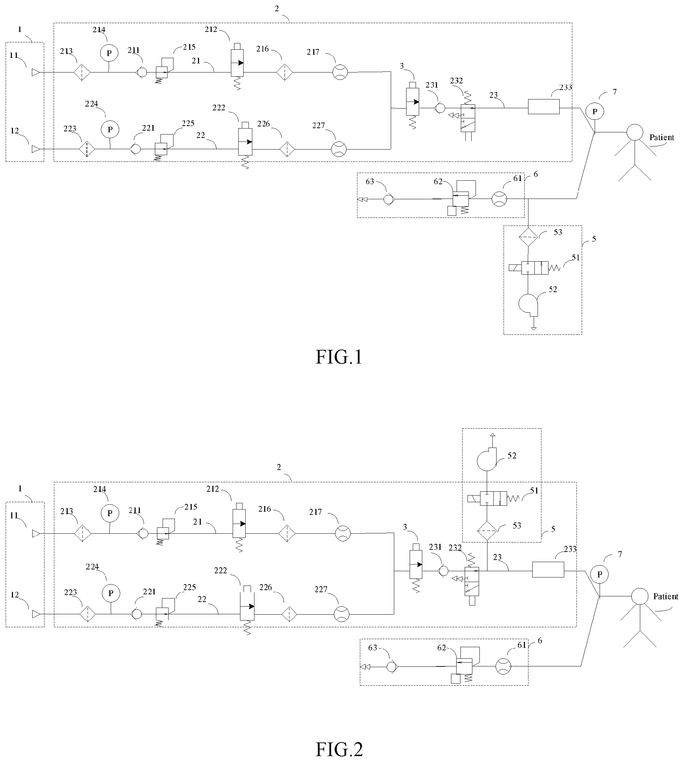

Embodiments of this disclosure provide a respiratory ventilation system. FIG. 1 is a first structural schematic block diagram of a respiratory ventilation system provided by an embodiment of this disclosure. As shown in FIG.1, the respiratory ventilation system includes a gas source interface 1, an inspiratory branch 2, a high-frequency oscillation generation device 3, a ventilation control device 4 (unshown), and an active expiratory device 5.

The inspiratory branch 2 is respectively connected with the gas source interface 1 and a patient pipeline which is connected with a respiratory system of a patient.

The high-frequency oscillation generation device 3 is configured to generate high-frequency oscillation for a gas of the inspiratory branch 2.

The ventilation control device 4 is connected with the inspiratory branch 2, the high-frequency oscillation generation device 3 and the active expiratory device 5, and is configured to control the high-frequency oscillation generation device 3 to generate the high-frequency oscillation for the gas of the inspiratory branch 2 according to a preset high-frequency oscillation frequency, and output a high-frequency oscillation gas which is generated by the high-frequency oscillation generation device 3 through the inspiratory branch 2 and the patient pipeline during an inspiratory phase, and is configured to control the active expiratory device 5 to actively extract, according to the preset high-frequency oscillation frequency, a gas which is exhaled by the patient through the patient pipeline during an expiratory phase.

It should be noted that in the embodiment of this disclosure, the inspiratory branch 2 of the respiratory ventilation system is configured to provide a gas delivery path during the inspiratory phase.

It should be noted that in the embodiment of this disclosure, as shown in FIG. 1, the high-frequency oscillation generation device 3 can be a high-frequency inspiratory valve. Specifically, the high-frequency inspiratory valve can be any one of proportional solenoid valve, blocking valve, servo valve and turbine. Of course, the high-frequency oscillation generation device 3 can also be other devices that can realize high-frequency oscillation of gas, and the embodiment of this disclosure makes no limitation for this.

It should be noted that in the embodiment of this disclosure, the medical staff can determine the preset high-frequency oscillation frequency according to the actual ventilation needs of patient. The specific preset high-frequency oscillation frequency can be 3-50HZ, and the embodiment of this disclosure makes no limitation for this.

It can be understood that in the embodiment of this disclosure, the high-frequency oscillation generation device 3 is arranged at the inspiratory branch 2, and the ventilation control device 4 is connected with the inspiratory branch 2 and the high-frequency oscillation generation device 3, so that during the inspiratory phase, the ventilation control device 4 can control the high-frequency oscillation generation device 3 to generate high-frequency oscillation for the gas of the inspiratory branch 2 according to the preset high-frequency oscillation frequency. Then, since the inspiratory branch 2 is also connected with the patient pipeline, the high-frequency oscillation gas, which is generated by the high-frequency oscillation generation device 3, flows through the inspiratory branch and is transmitted from the patient pipeline to the patient to realize the high-frequency inhalation of the patient.

It can be understood that in the embodiment of this disclosure, the ventilation control device 4 is also connected with the active expiratory device 5, so that during the expiratory phase, the active expiratory device 5 is controlled to actively extract the exhaled gas of the patient according to the preset high-frequency oscillation frequency, so as to realize active exhalation.

Specifically, in the embodiment of this disclosure, as shown in FIGS.1-2, the active expiratory device 5 includes a high-frequency valve 51 and/or an electric gas extraction device 52, the respiratory ventilation system further includes an expiratory branch 6 which is connected with the patient pipeline to discharge the exhaled gas of the patient. The active expiratory device 5 is arranged at the inspiratory branch 2 or the expiratory branch 6.

It can be understood that in the embodiment of this disclosure, the respiratory ventilation system also includes an expiratory branch 6 which is configured to provide an expiratory path during the expiratory phase.

It should be noted that in the embodiment of this disclosure, the high-frequency valve 51 included in the active expiratory device 5 can be a switching valve or a proportional valve. The electric gas extraction device 52 can be a turbine and other devices. During the expiratory phase, the ventilation control device 4 can control rotation of the turbine based on the preset high-frequency oscillation frequency. By controlling a rotation speed of the turbine, a negative force can be controlled to generate active exhalation. The turbine actively extracts the exhales gas of the patient and generates active exhalation. The specific high-frequency valve 51 and electric gas extraction device 52 can be selected according to the actual situation, and the embodiment of this disclosure makes no limitation for this.

In the embodiment of this disclosure, as shown in FIG.1, the active expiratory device 5 can be arranged at the expiratory branch 6 of the respiratory ventilation system. As shown in FIG.2, the active expiratory device 5 can also be arranged at the inspiratory branch 2 of the respiratory ventilation system. In addition, as shown in FIGS. 1 and 2, the active expiratory device includes not only the high-frequency valve 51 and the electric gas extraction device 52, but also an expiratory filter 53. During the expiratory phase, the ventilation control device 4 can control the high-frequency valve 51 to open, and control the rotation speed of the electric gas extraction device 52, such as the turbine, by regulating current or voltage, so as to extract the exhaled gas of the patient through the exhalation filter 53. In addition, the ventilation control device 4 can also control the expiratory valve of the expiratory branch 6 to open and discharge gas at the same time, so as to assist the electric gas extraction device 52 to discharge gas together.

It should be noted that in the embodiment of this disclosure, according to different actual situations, the high-frequency valve 51 can only be opened to realize active exhalation, the electric gas extraction device 52 can only be used to realize active exhalation, or both of the high-frequency valve 51 and the electric gas extraction device 52 can also be used to realize active exhalation at the same time. The embodiment of this disclosure makes no limitation for this.

Specifically, in the embodiment of this disclosure, the ventilation control device 4 is further configured to control the high-frequency valve 51 to open, and control an opening size of the high-frequency valve 51 and extraction power of the electric gas extraction device 52 according to the preset high-frequency oscillation frequency, during the expiratory phase, so as to actively extract the exhaled gas of the patient through the patient pipeline.

Optionally, the ventilation control device 4 is further configured to regulate an opening size of the high-frequency valve 51 according to the preset high-frequency oscillation frequency during the expiratory phase, so as to actively extract the exhaled gas of the patient through the patient pipeline.

Optionally, the ventilation control device 4 is further configured to regulate extraction power of the electric gas extraction device 52 according to the preset high-frequency oscillation frequency, during the expiratory phase, so as to actively extract the exhaled gas of the patient through the patient pipeline.

It should be noted that in the embodiment of this disclosure, when the high-frequency valve 51 is a proportional valve, the ventilation control device 4 can control the opening size of the proportional valve by regulating the current or voltage according to preset relevant parameters and actual demands of active exhalation. For example, during the expiratory phase, when the preset high-frequency oscillation frequency regulation requires a rapid decline in airway pressure, the ventilation control device 4 can adopt a faster valve opening control speed. When the preset high-frequency oscillation frequency regulation requires the airway pressure to fall to a larger negative pressure, the ventilation control device 4 can regulate the opening size of the proportional valve to a larger value. In addition, when the preset low-pressure oscillation value is not enough to start up the electric gas extraction device 52, the ventilation control device 4 can regulate the opening size of the proportional valve according to the numerical value of the low-pressure oscillation value. In some cases, the ventilation control device 4 needs to reduce the opening size of the proportional valve to satisfy the needs of the ventilation setting.

It should be noted that in the embodiment of this disclosure, when the high-frequency valve 51 and the electric gas extraction device 52 are combined to realize active exhalation according to the preset high-frequency oscillation frequency and the high-frequency valve 51 is a proportional valve, since the electric gas extraction device 52, such as the turbine, has a slow regulation response, the opening size of the proportional valve can also be regulated to bring its advantages of short response time and fast response into full play, so as to quickly reach the required pressure. In addition, the regulation resolution of the proportional valve is generally higher than that of the turbine, which can achieve more accurate ventilation control.

It should be noted that when the high-frequency valve 51 is composed of a fixed hole structure, the ventilation control device 4 cannot control its switching and opening size. Therefore, when the ventilation control device 4 controls the active expiratory device 5 to realize active exhalation, the expiratory valve always assists in ventilation.

It should be noted that in the embodiment of this disclosure, when the high-frequency valve 51 is a switching valve, the ventilation control device 4 cannot regulate its opening size, and the low-pressure oscillation value cannot be controlled without starting up the electric gas extraction device 52.

It should be noted that in the embodiment of this disclosure, the active expiratory device 5 may not include the high-frequency valve 51, but only include the electric gas extraction device 52. During the inspiratory phase, the ventilation control device 4 can minimize the leakage of gas from the electric gas extraction device 52 by regulating rotation speed or rotation direction of the electric gas extraction device 52, such as the turbine.

Specifically, in the embodiment of this disclosure, the ventilation control device 4 is also configured to control the active expiratory device 5 to be switched off, so as to discharge the exhaled gas of the patient through the patient pipeline and the expiratory branch 6.

It should be noted that in the embodiment of this disclosure, as shown in FIGS. 1 and 2, the expiratory branch 6 may include an expiratory flow sensor 61, an expiratory valve 62, and an expiratory check valve 63. The expiratory flow sensor 61 is connected with the patient pipeline to monitor the flow volume and tidal volume of the exhaled gas of the patient. The expiratory valve 62 is connected with the expiratory flow sensor 61 to control expiratory end pressure of the exhaled gas of the patient, and prevent alveoli collapse of the patient after exhalation. The expiratory check valve 63 is connected with the expiratory valve 62 to prevent gas from entering from the expiratory branch.

It should be noted that in the embodiment of this disclosure, during the expiratory phase, when normal frequency ventilation is adopted, the ventilation control device 4 controls the high-frequency valve 51 to close, and the exhaled gas of the patient passes through the expiratory flow sensor 61 of the expiratory branch 6 and is discharged through the expiratory valve 62.

It can be understood that in the embodiment of this disclosure, the actual state of the patient may not need to realize active exhalation during the expiratory phase. Therefore, the ventilation control device 4 can also control the active expiratory device 5 to be switched off during the expiratory phase, so that the exhaled gas of the patient from the patient pipeline can be discharged through the expiratory branch 6.

In the embodiment of this disclosure, as shown in FIGS. 1 and 2, the gas source interface 1 includes a first gas source interface 11 and a second gas source interface 12, the inspiratory branch 2 includes a first gas supply branch 21, a second gas supply branch 22 and a third gas supply branch 23.

A gas outlet end of the first gas supply branch 21 and a gas outlet end of the second gas supply branch 22 are respectively connected with a gas inlet end of the third gas supply branch 23.

A gas outlet end of the third gas supply branch 23 is connected with the patient pipeline.

A gas inlet end of the first gas supply branch 21 is connected with the first gas source interface 11.

A gas inlet end of the second gas supply branch 22 is connected with the second gas source interface 12.

It should be noted that in the embodiment of this disclosure, as shown in FIGS. 1 and 2, the first gas source interface 11 is connected with oxygen source, and the second gas source interface 12 is connected with air source. Of course, the first gas source interface 11 can also be connected with the air source. Accordingly, the second gas source interface 12 can be connected with the oxygen source. The embodiment of this disclosure makes no limitations for this.

Specifically, in the embodiment of this disclosure, as shown in FIGS. 1 and 2, the first gas supply branch 21 includes a first inspiratory check valve 211 and a first flow regulation valve 212 which are connected in sequence, the second gas supply branch 22 includes a second inspiratory check valve 221 and a second flow regulation valve 222 which are connected in sequence, and the third gas supply branch 23 includes a third inspiratory check valve 231.

The first inspiratory check valve 211 is connected with the first gas source interface 11, and the second inspiratory check valve 221 is connected with the second gas source interface 12.

It should be noted that in the embodiment of this disclosure, as shown in FIGS. 1 and 2, the first gas supply branch 21 can include not only the first inspiratory check valve 211 and the first flow regulation valve 212, but also a first filter 213, a first pressure sensor 214, a first pressure regulation valve 215, a second filter 216 and a first flow sensor 217. In addition, the second gas supply branch can include not only the second inspiratory check valve 221 and the second flow regulation valve 222, but also a third filter 223, a second pressure sensor 224, a second pressure regulation valve 225, a fourth filter 226 and a second flow sensor 227.

Specifically, in the embodiment of this disclosure, as shown in FIGS. 1 and 2, at the first gas supply branch 21, the first filter 213 is connected with the first gas source interface 11 to prevent impurities from flowing into downstream of the gas path to protect downstream devices. The first pressure sensor 214 is connected with the first filter 213 to monitor pressure of oxygen which is inputted from the first gas source interface 11, so as to realize an alarm indication when the pressure exceeds a maximum threshold or is lower than a minimum threshold. The first inspiratory check valve 211 is connected with the first pressure sensor 214 to prevent air from entering the branch, and to prevent air which enters the second gas supply branch 2 from reverse leakage when only the second gas supply branch 22 is opened. The first pressure regulation valve 215 is connected with the first inspiratory check valve 211, which can stabilize the input pressure of the gas source and ensure the accurate control of the downstream flow volume and pressure. The first flow regulation valve 212 is connected with the first pressure regulation valve 215 for regulating and controlling the oxygen flow. The second filter 216 is connected with the first flow regulation valve 212 and the first flow sensor 217. The second filter is further configured to purify inputted oxygen, protect the downstream first flow sensor 217 to accurately and smoothly measure the oxygen, and also play a role in stabilizing the flow rate.

Specifically, in the embodiment of this disclosure, as shown in FIGS. 1 and 2, at the second gas supply branch 22, the third filter 223 is connected with the second gas source interface 12 to prevent impurities from flowing into downstream of the gas path to protect the downstream devices. The second pressure sensor 224 is connected with the third filter 223 to monitor pressure of air which is inputted from the second gas source interface 12, so as to realize an alarm indication when the pressure exceeds a maximum threshold or is lower than a minimum threshold. The second inspiratory check valve 221 is connected with the second pressure sensor 224 to prevent oxygen from entering the branch, and to prevent air which enters the first gas supply branch 21 from reverse leakage when only the first gas supply branch 21 is opened. The second pressure regulation valve 225 is connected with the second inspiratory check valve 221, which can stabilize the input pressure of the gas source and ensure the accurate control of the downstream flow volume and pressure. The second flow regulation valve 222 is connected with the second pressure regulation valve 225 for regulating and controlling the air flow. The fourth filter 226 is connected with the second flow regulation valve 222 and the second flow sensor 227. The fourth filter 226 is further configured to purify inputted air, protect the downstream second flow sensor 227 to accurately and smoothly measure the air flow, and also play a role in stabilizing the flow rate.

It can be understood that in the embodiment of this disclosure, the first flow regulation valve 212 and the second flow regulation valve 222 control flow amount of oxygen and air respectively, so that when oxygen and air are mixed in the third gas supply branch 23 to obtain a mixed gas, the oxygen concentration of the mixed gas is controlled to satisfy the ventilation needs of different patients.

It should be noted that in the embodiment of this disclosure, as shown in FIGS. 1 and 2, the high-frequency oscillation generation device 3 is connected with the third gas supply branch 23, which includes not only a third inspiratory check valve 231, but also a safety valve 232 and a humidifier 233.

Specifically, in the embodiment of this disclosure, as shown in FIGS. 1 and 2, the high-frequency oscillation generation device 3 is sequentially connected with the third inspiratory check valve 231, and the first flow regulation valve 212 and the second flow regulation valve 222 can be respectively connected with the high-frequency oscillation generation device 3. In this application document, connection includes direct connection and indirect connection. The first flow regulation valve 212 and the second flow regulation valve 222 can be directly connected with the high-frequency oscillation generation device 3, respectively. Of course, the first flow regulation valve 212 and the second flow regulation valve 222 can also be indirectly connected with the high-frequency oscillation generation device 3, respectively. For example, the first flow regulation valve 212 may be indirectly connected with the high-frequency oscillation generation device 3 via the second filter 216 and/or the first flow sensor 217. The second flow regulation valve 222 may be indirectly connected with the high-frequency oscillation generation device 3 via the fourth filter 226 and/ or the second flow sensor 227.

It should be noted that in the embodiment of this disclosure, as shown in FIGS. 1 and 2, at the third gas supply branch 23, the high-frequency oscillation generation device 3 can be configured to regulate the flow volume of the mixed gas of oxygen and air, and control the airway pressure according to the flow volume of the outputted mixed gas, in which the airway pressure is monitored by a proximal pressure sensor 7. The third inspiratory check valve 231 is connected with the high-frequency oscillation generation device 3 to prevent the exhaled gas from entering the high-frequency oscillation generation device 3 and its upstream devices of the third gas supply branch 23 when the patient exhales during the expiratory phase. The safety valve 232 is connected with the third inspiratory check valve 231. When the pressure of the mixed gas reaches a maximum preset value during the inspiratory process, the safety valve 232 opens to discharge the gas and achieves the purpose of pressure discharge. In addition, during the expiratory phase, if the expiratory branch and the active expiratory device 5 fail and cannot discharge the exhaled gas normally, the safety valve 232 can also be opened to discharge the exhaled gas. Inside the third gas supply branch 23, when the mixed gas sent to the front end of the safety valve 232 is not enough, the safety valve 232 can also be switched to the atmosphere to suck gas from the atmosphere to make up for the deficiency. The humidifier 233 is connected with the safety valve 232, which can heat and humidify the incoming mixed gas and control the temperature and humidity of the mixed gas, so as to ensure the inspiratory comfort of patient.

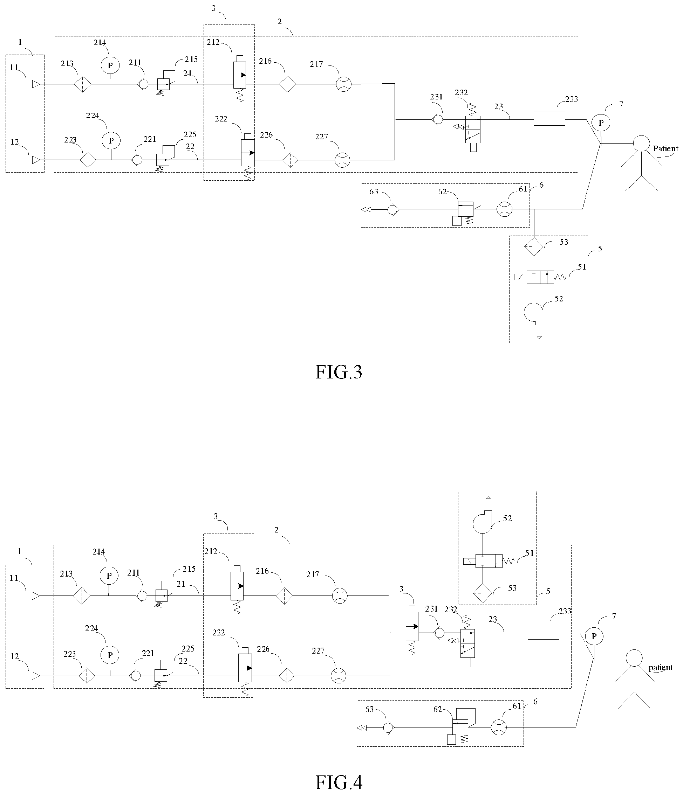

FIG. 3 is a third structural schematic block diagram of a respiratory ventilation system provided by an embodiment of this disclosure. FIG. 4 is a fourth structural schematic block diagram of a respiratory ventilation system provided by an embodiment of this disclosure. As shown in FIGS. 3 and 4, in the embodiment of this disclosure, the high-frequency oscillation generation device 3 may be composed of a first flow regulation valve 212 and a second flow regulation valve 222.

It should be noted that in the embodiment of this disclosure, as shown in FIGS. 3 and 4, when the first flow regulation valve 212 and the second flow regulation valve 222 form a high-frequency oscillation generation device, that is, the ventilation control device 4 is connected with the first flow regulation valve 212 and the second flow regulation valve 222, and the ventilation control device 4 can control the first flow regulation valve 212 and the second flow regulation valve 222 according to the preset high-frequency oscillation frequency, such that the gas at the corresponding gas supply branch generates high-frequency oscillation. Further, the mixing of the two gases is realized in the third gas supply branch, and finally transmitted to the patient through the patient pipeline.

It should be noted that in the embodiment of this disclosure, as shown in FIGS.1-4, a proximal pressure sensor 7 can be arranged at the patient pipeline to monitor the proximal pressure of the patient in real time and feed it back to the ventilation control device 4 for relevant control by the ventilation control device 4.

It should be noted that in the embodiment of this disclosure, as shown in FIGS.1 and 3, the active expiratory device 5 is specifically connected with the expiratory branch 6. In addition, as shown in FIGS. 2 and 4, the active expiratory device 5 can also be connected with an outlet of the third inspiratory check valve 231.

Specifically, in the embodiment of this disclosure, the ventilation control device 4 controls the active expiratory device 5 and the high-frequency oscillation generation device 3 work together to realize the control of the average airway pressure.

It can be understood that in the embodiment of this disclosure, during the inspiratory phase, the ventilation control device 4 controls the high-frequency oscillation generation device 3 to quickly regulate according to the preset high-frequency oscillation frequency, so that the gas of the inspiratory branch 2 generates high-frequency oscillation and controls peak pressure of each cycle. In addition, during the inspiratory phase, the ventilation control device 4 can also control the active expiratory device 5 to extract part of the gas from the inspiratory branch 2, so as to control the average airway pressure to reach the preset state. Among them, the proximal pressure sensor 7 can monitor the pressure and feed it back to the ventilation control device 4 in time. According to the feedback pressure, the ventilation control device 4 controls the active expiratory device 5 to extract gas.

It should be noted that in the embodiment of this disclosure, the ventilation control device 4 can also control the active expiratory device 5 and the exhalation valve 62 of the exhalation branch 6 at the same time to work together with the high-frequency oscillation generation device 3 to control the average airway pressure.

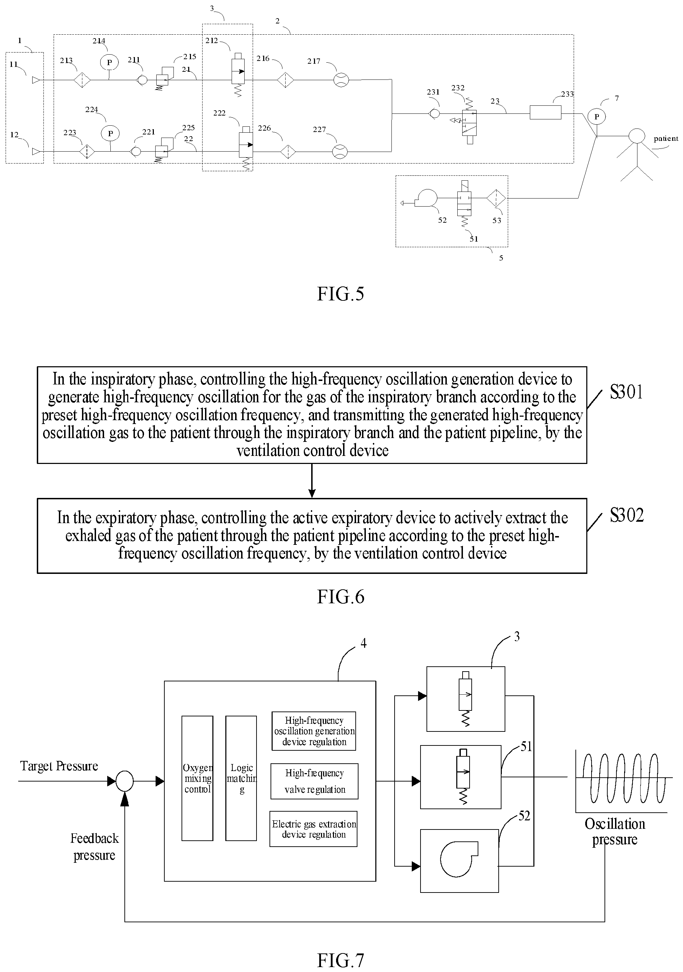

FIG. 5 is a fifth structural schematic block diagram of a respiratory ventilation system provided by an embodiment of this disclosure. As shown in FIG. 5, in the embodiment of this disclosure, the expiratory branch 6 can also be excluded from the respiratory ventilation system. Not only the active expiratory function can be realized by the active expiratory device 5, but also the passive expiratory function of the expiratory branch 6 can be realized by the active expiratory device 5, under the control of the ventilation control device 4. The embodiment of this disclosure makes no limitation for this.

Embodiments of this disclosure disclose a respiratory ventilation system which includes a gas source interface, an inspiratory branch, a high-frequency oscillation generation device, a ventilation control device, and an active expiratory device; the inspiratory branch is respectively connected with the gas source interface and a patient pipeline which is connected with a respiratory system of a patient; the high-frequency oscillation generation device is configured to generate high-frequency oscillation for a gas of the inspiratory branch; the ventilation control device is connected with the inspiratory branch, the high-frequency oscillation generation device and the active expiratory device, and is configured to control the high-frequency oscillation generation device to generate the high-frequency oscillation for the gas of the inspiratory branch according to a preset high-frequency oscillation frequency during an inspiratory phase, output a high-frequency oscillation gas which is generated by the high-frequency oscillation generation device through the inspiratory branch and the patient pipeline, and control the active expiratory device to actively extract, according to the preset high-frequency oscillation frequency, a gas which is exhaled by the patient through the patient pipeline during an expiratory phase. The respiratory ventilation system provided by embodiments of this disclosure uses the active expiratory device to extract the exhaled gas of the patient to produce active exhalation, so as to support the high-frequency ventilation mode, which not only has a smaller system volume, but also reduces gas consumption and noise.

Embodiment of this disclosure discloses a respiratory ventilation method which is implemented by the above respiratory ventilation system. FIG. 6 is a flow diagram of a respiratory ventilation method provided by an embodiment of this disclosure. As shown in FIG.6, the respiratory ventilation method mainly includes following steps.

In step S601, during the inspiratory phase, the high-frequency oscillation generation device is controlled to generate high-frequency oscillation for the gas of the inspiratory branch according to the preset high-frequency oscillation frequency, and the generated high-frequency oscillation gas is transmitted to the patient through the inspiratory branch and the patient pipeline, by the ventilation control device.

In the embodiment of this disclosure, during the inspiratory phase, the respiratory ventilation system can control the high-frequency oscillation generation device 3 through the ventilation control device 4 to generate high-frequency oscillation for the gas of the inspiratory branch 2 according to the preset high-frequency oscillation frequency, and transmit the generated high-frequency oscillation gas to the patient through the inspiratory branch 2 and the patient pipeline.

It should be noted that in the embodiment of this disclosure, during the inspiratory phase, the ventilation control device 4 can generate corresponding operation instructions according to the preset high-frequency oscillation frequency and transmit them to the high-frequency oscillation generation device 3. The high-frequency oscillation generation device 3 can execute the received operation instructions to realize the high-frequency oscillation of the gas of the inspiratory branch 2.

It should be noted that in the embodiment of this disclosure, the preset high-frequency oscillation frequency can be determined according to the actual requirements of the patient, and the embodiment of this disclosure makes no limitation for this.

It should be noted that in the embodiment of this disclosure, the respiratory ventilation system can also control the active expiratory device 5 and the high-frequency oscillation generation device 3 to work together through the ventilation control device 4, so as to realize the control of the average airway pressure.

Specifically, during the inspiratory phase, the ventilation control device 4 can control the active expiratory device 5 to work, to extract part of the gas from the inspiratory branch 2, so as to control the average airway pressure to reach the preset state. Among them, the proximal pressure sensor 7 at the patient pipeline can monitor the pressure and feed it back to the ventilation control device 4 in time. According to the feedback pressure, the ventilation control device 4 controls the active expiratory device 5 to extract gas.

In step S602, during the expiratory phase, the active expiratory device is controlled to actively extract the exhaled gas of the patient through the patient pipeline according to the preset high-frequency oscillation frequency, by the ventilation control device.

In the embodiment of this disclosure during the expiratory phase, the respiratory ventilation system can also control the active expiratory device 5 to actively extract the exhaled gas of the patient through the patient pipeline according to the preset high-frequency oscillation frequency, by the ventilation control device 4.

Specifically, in the embodiment of this disclosure, the active expiratory device 5 includes a high-frequency valve 51 and/or an electric gas extraction device 52; wherein during the expiratory phase, the respiratory ventilation system can also control the active expiratory device 5 to actively extract the exhaled gas of the patient through the patient pipeline according to the preset high-frequency oscillation frequency, by the ventilation control device 4, which includes followings. During the expiratory phase, the respiratory ventilation system can control the high-frequency valve 51 to open, and control an opening size of the high-frequency valve 51 and extraction power of the electric gas extraction device 52 according to the preset high-frequency oscillation frequency, by the ventilation control device 4, so as to actively extract the exhaled gas of the patient through the patient pipeline. Alternatively, the respiratory ventilation system can, during the expiratory phase, regulate an opening size of the high-frequency valve 51 according to the preset high-frequency oscillation frequency by the ventilation control device 4, so as to actively extract the exhaled gas of the patient through the patient pipeline. Alternatively, the respiratory ventilation system can, during the expiratory phase, regulate extraction power of the electric gas extraction device 52 according to the preset high-frequency oscillation frequency by the ventilation control device 4, so as to actively extract the exhaled gas of the patient through the patient pipeline.

It should be noted that in the embodiment of this disclosure, the ventilation control device 4 can control the peak pressure and minimum pressure at the patient end of each cycle, as well as the oxygen concentration of the inhaled gas and other related parameters to achieve the target parameters in the process of controlling the corresponding devices for rapid regulation according to the preset high-frequency oscillation frequency.

FIG. 7 is a detailed control diagram of an exemplary ventilation control device provided by an embodiment of this disclosure. As shown in FIG.7, in the process of high-frequency ventilation, the ventilation control device 4 is mainly responsible for algorithm operation and control operation of core devices such as the high-frequency oscillation generation device 3 and the active expiratory device 5, including the high-frequency valve 51 and the electric gas extraction device 52. The specific functions of ventilation control device 4 mainly include high-frequency oxygen mixing control, logic matching of control devices, regulation of high-frequency oscillation generation device, regulation of high-frequency valve, and regulation of electric gas extraction device. Among them, the function of high-frequency oxygen mixing is mainly to regulate the flow rate for achieving the desired oxygen concentration target. The logic matching function is mainly to allocate and coordinate the functions of high-frequency oscillation generation device 3, high-frequency valve 51 and electric gas extraction device 52 in the process of pressure control, so as to achieve optimal control coordination. For example, during the expiratory phase, the ventilation control device 4 preferentially switches off the high-frequency oscillation generation device 3 to reduce the pressure. If the switching-off of the high-frequency oscillation generation device cannot satisfy the pressure reduction demand, then the high-frequency valve 51 and the electric gas extraction device 52 are switched on to reduce the pressure. The regulation function of high-frequency oscillation generation device 3 is mainly responsible for the closed-loop feedback control of flow rate of high-frequency oscillation generation device 3 during the pressure oscillation. The regulation function of high-frequency valve is mainly responsible for the control of the opening size and opening speed of high-frequency valve 51. When the pressure is required to drop rapidly, the high-frequency valve 51 adopts a faster opening control speed. When the pressure is required to drop to a larger negative pressure, the high-frequency valve 51 is regulated to a larger opening size. The regulation function of the electric gas extraction device is mainly responsible for the closed-loop control of rotation speed of the electric gas extraction device 52, such as the turbine, to achieve a stable output. Finally, the high-frequency oscillation generation device 3, the high-frequency valve 51 and the electric gas extraction device 52 execute the corresponding operation instructions according to a target regulation value to achieve the desired control effect.

It should be noted that in the embodiment of this disclosure, as shown in FIG. 7, during the high-frequency oscillation ventilation, the monitoring of oscillation related parameters, such as pressure or flow rate, can be obtained and fed back to the ventilation control device 4 through the sensor which is arranged at the corresponding position in the respiratory ventilation system.

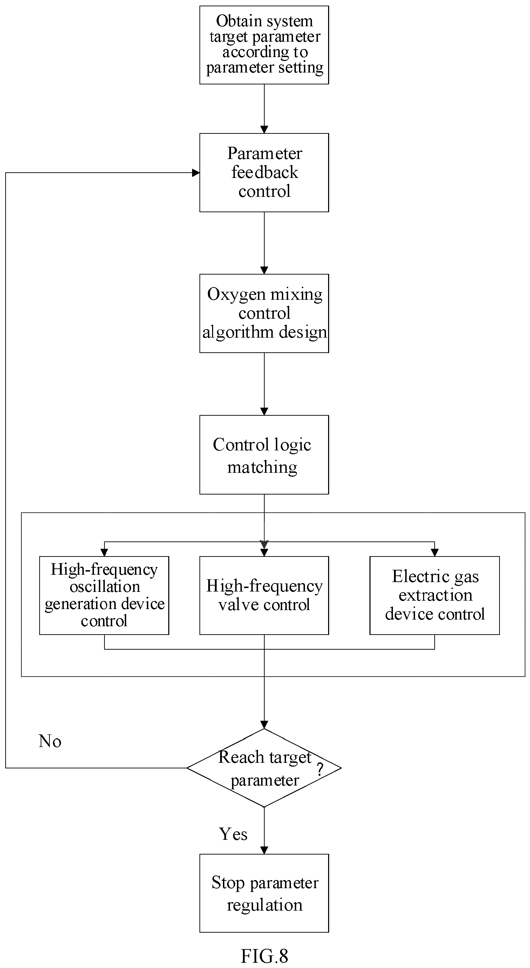

FIG. 8 is a control flow diagram of an exemplary ventilation control device provided by an embodiment of this disclosure. As shown in FIG. 8, the ventilation control device 4 firstly obtains a target parameter according to system parameter setting, such as target control pressure, and carries out pressure closed-loop feedback control according to an actual flow rate at the patient end. Then, the ventilation control device 4 carries out oxygen concentration feedback control according to total target flow rate which is obtained by the parameter feedback and preset target oxygen concentration. At the same time, the logic matching function carries out control logic matching based on control states of the high-frequency oscillation generation device 3, the high-frequency valve 51 and the electric gas extraction device 52. Then the high-frequency oscillation generation device 3, the high-frequency valve 51 and the electric gas extraction device 52 regulate oscillation pressure and other related parameters according to control sequence and control strength of the logic matching. During the whole process of oscillation pressure regulation, the ventilation control device 4 monitors the actual pressure at the patient end in real time. If the target control pressure is reached, the pressure feedback is stopped and the current control state is maintained. Otherwise, the pressure closed-loop feedback control is continued.

The embodiment of this disclosure provides a respiratory ventilation method, which includes: during the inspiratory phase, controlling the high-frequency oscillation generation device to generate the high-frequency oscillation for the gas of the inspiratory branch according to the preset high-frequency oscillation frequency, and transmitting the generated high-frequency oscillation gas to the patient through the inspiratory branch and the patient pipeline, by the ventilation control device; during the expiratory phase, controlling the active expiratory device to actively extract the exhaled gas of the patient through the patient pipeline according to the preset high-frequency oscillation frequency, by the ventilation control device. The respiratory ventilation method provided by the embodiment of this disclosure uses the active expiratory device to extract the exhaled gas of the patient to produce active exhalation, so as to support the high-frequency ventilation mode, which not only has a small system volume, but also reduces gas consumption and noise.

The above are only preferred embodiments of this disclosure and are not used to limit the scope of protection of this disclosure.

### INDUSTRIAL APPLICABILITY

In the technical scheme of the embodiment of this disclosure, during the inspiratory phase, the high-frequency oscillation generation device is controlled to generate high-frequency oscillation for the gas of the inspiratory branch according to the preset high-frequency oscillation frequency, and the generated high-frequency oscillation gas is transmitted to the patient through the inspiratory branch and the patient pipeline, by the ventilation control device; during the expiratory phase, the active expiratory device is controlled to actively extract the exhaled gas of the patient through the patient pipeline according to the preset high-frequency oscillation frequency, by the ventilation control device. The technical scheme provided by the embodiment of this disclosure uses the active expiratory device to extract the exhaled gas of the patient to produce active exhalation, so as to support the high-frequency ventilation mode, which not only has a small system volume, but also reduces gas consumption and noise.

## Claims

1. A respiratory ventilation system, comprising:
a gas source interface (1), an inspiratory branch (2), a high-frequency oscillation generation device (3), and a ventilation control device (4);
the inspiratory branch (2) is respectively connected with the gas source interface (1) and a patient pipeline which is connected with a respiratory system of a patient;
the high-frequency oscillation generation device (3) is configured to generate high-frequency oscillation for a gas of the inspiratory branch (2);
**characterized in that**, the respiratory ventilation system further comprises an active expiratory device (5), wherein the active expiratory device (5) comprises an electric gas extraction device (52);
the ventilation control device (4) is connected with the inspiratory branch (2), the high-frequency oscillation generation device (3) and the active expiratory device (5), wherein the ventilation control device (4) is configured to control the high-frequency oscillation generation device (3) to generate the high-frequency oscillation for the gas of the inspiratory branch (2) according to a preset high-frequency oscillation frequency and to output a high-frequency oscillation gas which is generated by the high-frequency oscillation generation device (3) through the inspiratory branch (2) and the patient pipeline during an inspiratory phase, and is also configured to control the active expiratory device (5) to actively extract, according to the preset high-frequency oscillation frequency, a gas which is exhaled by the patient through the patient pipeline during an expiratory phase.

2. The respiratory ventilation system according to claim 1, **characterized in that**, the active expiratory device (5) further comprises a high-frequency valve (51);
the respiratory ventilation system further comprises an expiratory branch (6) which is connected with the patient pipeline to discharge the exhaled gas of the patient;
the active expiratory device (5) is arranged at the inspiratory branch (2) or the expiratory branch (6).

3. The respiratory ventilation system according to claim 2, **characterized in that**, the high-frequency valve is a switching valve or a proportional valve.

4. The respiratory ventilation system according to claim 2, **characterized in that**, the ventilation control device (4) is further configured to control the active expiratory device (5) to be switched off, so as to discharge the exhaled gas of the patient through the patient pipeline and the expiratory branch (6).

5. The respiratory ventilation system according to claim 2, **characterized in that**,
during the expiratory phase, the ventilation control device (4) is further configured to, control the high-frequency valve to open, and control an opening size of the high-frequency valve and extraction power of the electric gas extraction device (52) according to the preset high-frequency oscillation frequency, so as to actively extract the exhaled gas of the patient through the patient pipeline; or
during the expiratory phase, the ventilation control device (4) is further configured to, regulate an opening size of the high-frequency valve according to the preset high-frequency oscillation frequency, so as to actively extract the exhaled gas of the patient through the patient pipeline; or
during the expiratory phase, the ventilation control device (4) is further configured to, regulate extraction power of the electric gas extraction device (52) according to the preset high-frequency oscillation frequency, so as to actively extract the exhaled gas of the patient through the patient pipeline.

6. The respiratory ventilation system according to claim 1, **characterized in that**, the gas source interface comprises a first gas source interface (11) and a second gas source interface (12), the inspiratory branch (2) comprises a first gas supply branch (21), a second gas supply branch (22) and a third gas supply branch (23);
wherein a gas outlet end of the first gas supply branch (11) and a gas outlet end of the second gas supply branch (12) are respectively connected with a gas inlet end of the third gas supply branch (23);
a gas outlet end of the third gas supply branch (23) is connected with the patient pipeline;
a gas inlet end of the first gas supply branch (11) is connected with the first gas source interface (11);
a gas inlet end of the second gas supply branch (12) is connected with the second gas source interface (12).

7. The respiratory ventilation system according to claim 6, **characterized in that**, the first gas supply branch (11) comprises a first inspiratory check valve (211) and a first flow regulation valve (212) which are connected in sequence, the second gas supply branch (12) comprises a second inspiratory check valve (221) and a second flow regulation valve (222) which are connected in sequence, and the third gas supply branch (23) comprises a third inspiratory check valve (231);
the first inspiratory check valve (211) is connected with the first gas source interface (11), and the second inspiratory check valve (221) is connected with the second gas source interface (12).

8. The respiratory ventilation system according to claim 7, **characterized in that**, the high-frequency oscillation generation device (3) and the third inspiratory check valve (231) are connected in sequence;
the first flow regulation valve (212) and the second flow regulation valve (222) are respectively connected with the high-frequency oscillation generation device (3).

9. The respiratory ventilation system according to claim 7, **characterized in that**, the high-frequency oscillation generation device (3) comprises the first flow regulation valve (212) and the second flow regulation valve (222).

10. The respiratory ventilation system according to claim 1, **characterized in that**, the ventilation control device (4) is configured to control, during the inspiratory phase, the active expiratory device (5) to work together with the high-frequency oscillation generation device (3) to, to extract part of the gas from the inspiratory branch (2), so as to control an average airway pressure.

11. The respiratory ventilation system according to claim 1, **characterized in that**, the high-frequency oscillation generation device (3) is a high-frequency inspiratory valve.

12. The respiratory ventilation system according to claim 11, **characterized in that**, the high-frequency inspiratory valve is any one of proportional solenoid valve, blocking valve, servo valve and turbine.

13. The respiratory ventilation system according to claim 7, **characterized in that**, the active expiratory device (5) is connected with an outlet of the third inspiratory check valve (231), or the active expiratory device (5) is connected with an expiratory branch (6).

## Patentansprüche

1. Ein Beatmungssystem mit:
einer Gasquellen-Bedienungseinheit (1), einem Inspirationsschenkel (2), einer HochfrequenzOszillations-Erzeugungsvorrichtung (3) und einer Beatmungssteuerungsvorrichtung (4);
wobei der Inspirationsschenkel (2) jeweils mit der Gasquellen- Bedienungseinheit (1) und einem Leitungssystem verbunden ist, das mit einem Atemsystem eines Patienten verbunden ist; und
die Hochfrequenzoszillations-Erzeugungsvorrichtung (3) so konfiguriert ist, dass sie Hochfrequenzoszillationen für ein Gas des Inspirationsschenkels (2) erzeugt;
**dadurch gekennzeichnet, dass** das Beatmungssystem ferner eine aktive Exspirationsvorrichtung (5) mit einer elektrischen Gasabsaugvorrichtung (52) umfasst;
dass die Beatmungssteuerungsvorrichtung (4) imit dem Inspirationsschenkel (2), der Hochfrequenzoszillations-Erzeugungsvorrichtung (3) und der aktiven Exspirationsvorrichtung (5) verbunden und so konfiguriert ist, dass sie die Hochfrequenzoszillations-Erzeugungsvorrichtung (3) steuert, um gemäß einer vorgegebenen Hochfrequenzoszillationsfrequenz die Hochfrequenzoszillation für das Gas des Inspirationsschenkels (2) zu erzeugen und während einer Inspirationsphase ein von der Hochfrequenzoszillations-Erzeugungsvorrichtung (3) erzeugtes Hochfrequenzoszillationsgas über den Inspirationsschenkel (2) und das Patientenleitungssystem auszugeben; sie ist auch so konfiguriert, dass sie die aktive Exspirationsvorrichtung (5) steuert, um entsprechend der vorgegebenen Hochfrequenzoszillationsfrequenz aktiv ein von dem Patienten durch das Patientenleitungssystem während einer Exspirationsphase ausgeatmetes Gas abzusaugen.

2. Das Beatmungssystem nach Anspruch 1 ist **dadurch gekennzeichnet, dass** die aktive Exspirationsvorrichtung (5) ferner mit einem Hochfrequenzventil (51) ausgestattet ist.
Das Beatmungssystem umfasst ferner einen Exspirationsschenkel (6), der mit dem Patientenleitungssystem verbunden ist, um das ausgeatmete Gas des Patienten abzuführen;
wobei die aktive Exspirationsvorrichtung (5) entweder am Inspirationsschenkel (2) oder am Exspirationsschenkel (6) angeordnet ist.

3. Das Beatmungssystem nach Anspruch 2 ist **dadurch gekennzeichnet, dass** das Hochfrequenzventil ein Umschaltventil oder ein Proportionalventil ist.

4. Das Beatmungssystem nach Anspruch 2 ist **dadurch gekennzeichnet, dass** die Beatmungssteuerungsvorrichtung (4) ferner so konfiguriert ist, dass sie die aktive Exspirationsvorrichtung (5) abschaltet, um das ausgeatmete Gas des Patienten durch das Patientenleitungssystem und den Exspirationsschenkel (6) abzuführen.

5. Das Beatmungssystem nach Anspruch 2 ist **dadurch gekennzeichnet, dass** die Beatmungssteuerungsvorrichtung (4) während der Exspirationsphase ferner konfiguriert ist, das Hochfrequenzventil zu öffnen und die Öffnungsgröße des Hochfrequenzventils sowie die Absaugleistung der elektrischen Gasabsaugvorrichtung (52) gemäß der vorgegebenen Hochfrequenzoszillationsfrequenz zu steue, um das ausgeatmete Gas des Patienten aktiv durch das Patientenleitungssystem abzusaugen; oder
die Öffnungsgröße des Hochfrequenzventils gemäß der vorgegebenen Hochfrequenzoszillationsfrequenz (4) zu regeln, um das ausgeatmete Gas des Patienten aktiv durch das Patientenleitungssystem abzusaugen; oder
die Absaugleistung der elektrischen Gasabsaugvorrichtung (52) gemäß der vorgegebenen Hochfrequenzoszillationsfrequenz (4) zu regeln, um das ausgeatmete Gas des Patienten aktiv durch das Patientenleitungssystem abzusaugen.

6. Das Beatmungssystem nach Anspruch 1 ist **dadurch gekennzeichnet, dass** die Gasquellen-Bedienungseinheit eine erste Gasquellen-Bedienungseinheit (11) und eine zweite Gasquellen-Bedienungseinheit (12) umfasst, wobei der Inspirationsschenkel (2) einen ersten Gasversorgungsbereich (21), einen zweiten Gasversorgungsbereich (22) und einen dritten Gasversorgungsbereich (23) umfasst;
wobei ein Gasaustrittsende des ersten Gasversorgungsbereichs (11) und ein Gasaustrittsende des zweiten Gasversorgungsbereichs (12) jeweils mit einem Gaseintrittsende des dritten Gasversorgungsbereichs (23) verbunden sind;
ein Gasaustrittsende des dritten Gasversorgungsbereichs (23) mit dem Patientenleitungssystem verbunden ist;
ein Gaseintrittsende des ersten Gasversorgungsbereichs (11) mit der ersten Gasquellen-Bedienungseinheit (11) verbunden ist;
ein Gaseintrittsende des zweiten Gasversorgungsbereichs (12) mit der zweiten Gasquellen-Bedienungseinheit (12) verbunden ist.

7. Das Beatmungssystem nach Anspruch 6 ist **dadurch gekennzeichnet, dass** der erste Gasversorgungsbereich (11) ein erstes Inspirationsrückschlagventil (211) und ein erstes Durchflussregelventil (212) umfasst, die in Reihe geschaltet sind; der zweite Gasversorgungbereich (12) ein zweites Inspirationsrückschlagventil (221) und ein zweites Durchflussregelventil (222) umfasst, die in Reihe geschaltet sind; und der dritte Gasversorgungsbereich (23) ein drittes Inspirationsrückschlagventil (231) umfasst;
wobei das erste Inspirationsrückschlagventil (211) mit der ersten Gasquellen-Bedienungseinheit (11) verbunden ist und das zweite Inspirationsrückschlagventil (221) mit der zweiten Gasquellen- Bedienungseinheit (12).

8. Das Beatmungssystem nach Anspruch 7 ist **dadurch gekennzeichnet, dass** die Hochfrequenzoszillations-Erzeugungsvorrichtung (3) und das dritte Inspirationsrückschlagventil (231) in Reihe geschaltet sind und
das erste Durchflussregelventil (212) und das zweite Durchflussregelventil (222) jeweils mit der Hochfrequenzoszillations-Erzeugungsvorrichtung (3) verbunden sind.

9. Das Beatmungssystem nach Anspruch 7 ist **dadurch gekennzeichnet, dass** die Hochfrequenzoszillations-Erzeugungsvorrichtung (3) das erste Durchflussregelventil (212) und das zweite Durchflussregelventil (222) umfasst.

10. Das Beatmungssystem nach Anspruch 1 ist **dadurch gekennzeichnet, dass** die Beatmungssteuerungsvorrichtung (4) während der Inspirationsphase so konfiguriert ist, dass sie die aktive Exspirationsvorrichtung (5) zusammen mit der Hochfrequenzoszillations-Erzeugungsvorrichtung (3) steuert, um einen Teil des Gases aus dem Inspirationsschenkel (2) abzusaugen und so einen durchschnittlichen Atemwegsdruck zu kontrollieren.

11. Das Beatmungssystem nach Anspruch 1 ist **dadurch gekennzeichnet, dass** die Hochfrequenzoszillations-Erzeugungsvorrichtung (3) ein Hochfrequenz-Inspirationsventil ist..

12. Das Beatmungssystem nach Anspruch 11 ist **dadurch gekennzeichnet, dass** das Hochfrequenz-Inspirationsventil entweder ein Proportional-Magnetventil, ein Sperrventil, ein Servoventil oder eine Turbine ist.

13. Das Beatmungssystem nach Anspruch 7 ist **dadurch gekennzeichnet, dass** die aktive Exspirationsvorrichtung (5) entweder mit einem Auslass des dritten Inspirationsrückschlagventils (231) oder mit einem Exspirationsschenkel (6) verbunden ist.

## Revendications

1. Système de ventilation respiratoire, comprenant :
une interface de source de gaz (1), une branche inspiratoire (2), un dispositif de génération d'oscillation haute fréquence (3), et un dispositif de commande de ventilation (4) ;
la branche inspiratoire (2) est respectivement reliée à l'interface de source de gaz (1) et à une conduite patient laquelle est reliée à un système respiratoire d'un patient ;
le dispositif de génération d'oscillation haute fréquence (3) est configuré pour générer une oscillation haute fréquence pour un gaz de la branche inspiratoire (2) ;
**caractérisé en ce que**, le système de ventilation respiratoire comprend en outre un dispositif expiratoire actif (5), où le dispositif expiratoire actif (5) comprend un dispositif d'extraction de gaz électrique (52) ;
le dispositif de commande de ventilation (4) est relié à la branche inspiratoire (2), au dispositif de génération d'oscillation haute fréquence (3) et au dispositif expiratoire actif (5), où le dispositif de commande ventilation (4) est configuré pour commander le dispositif de génération d'oscillation haute fréquence (3) pour générer l'oscillation haute fréquence pour le gaz de la branche inspiratoire (2) selon une fréquence d'oscillation haute fréquence préétablie et pour délivrer un gaz d'oscillation haute fréquence lequel est généré par le dispositif de génération d'oscillation haute fréquence (3) à travers la branche inspiratoire (2) et la conduite patient durant une phase inspiratoire, et est également configuré pour commander le dispositif expiratoire actif (5) pour extraire activement, selon la fréquence d'oscillation haute fréquence préétablie, un gaz qui est exhalé par le patient à travers la conduite patient durant une phase expiratoire.

2. Système de ventilation respiratoire selon la revendication 1, **caractérisé en ce que**, le dispositif expiratoire actif (5) comprend en outre une vanne haute fréquence (51) ;
le système de ventilation respiratoire comprend en outre une branche expiratoire (6) laquelle est reliée à la conduite patient pour évacuer le gaz exhalé du patient ;
le dispositif expiratoire actif (5) est agencé au niveau de la branche expiratoire (2) ou de la branche expiratoire (6).

3. Système de ventilation respiratoire selon la revendication 2, **caractérisé en ce que**, la vanne haute fréquence est une vanne de commutation ou une vanne proportionnelle.

4. Système de ventilation respiratoire selon la revendication 2, **caractérisé en ce que**, le dispositif de commande de ventilation (4) est en outre configuré pour commander le dispositif expiratoire actif (5) pour l'arrêter, de sorte à évacuer le gaz exhalé du patient à travers la conduite patient et la branche expiratoire (6).

5. Système de ventilation respiratoire selon la revendication 2, **caractérisé en ce que**,
durant la phase expiratoire, le dispositif de commande de ventilation (4) est en outre configuré pour commander la vanne haute fréquence de sorte à l'ouvrir, et commander une taille d'ouverture de la vanne haute fréquence et une puissance d'extraction du dispositif d'extraction de gaz électrique (52) selon la fréquence d'oscillation haute fréquence préétablie, de sorte à extraire activement le gaz exhalé du patient à travers la conduite patient ; ou
durant la phase expiratoire, le dispositif de commande de ventilation (4) est en outre configuré pour réguler une taille d'ouverture de la vanne haute fréquence selon la fréquence d'oscillation haute fréquence préétablie, de sorte à extraire activement le gaz exhalé du patient à travers la conduite patient ; ou
durant la phase expiratoire, le dispositif de commande de ventilation (4) est en outre configuré pour réguler une puissance d'extraction du dispositif d'extraction de gaz électrique (52) selon la fréquence d'oscillation haute fréquence préétablie, de sorte à extraire activement le gaz exhalé du patient à travers la conduite patient.

6. Système de ventilation respiratoire selon la revendication 1, **caractérisé en ce que**, l'interface de source de gaz comprend une première interface de source de gaz (11) et une seconde interface de source de gaz (12), la branche inspiratoire (2) comprend une première branche d'alimentation en gaz (21), une deuxième branche d'alimentation en gaz (22) et une troisième branche d'alimentation en gaz (23) ;
où une extrémité de sortie de gaz de la première branche d'alimentation en gaz (11) et une extrémité de sortie de gaz de la deuxième branche d'alimentation en gaz (12) sont respectivement reliées à une extrémité d'entrée de gaz de la troisième branche d'alimentation en gaz (23) ;
une extrémité de sortie de gaz de la troisième branche d'alimentation en gaz (23) est reliée à la conduite patient ;
une extrémité d'entrée de gaz de la première branche d'alimentation en gaz (11) est reliée à la première interface de source de gaz (11) ;
une extrémité d'entrée de gaz de la deuxième branche d'alimentation en gaz (12) est reliée à la seconde interface de source de gaz (12).

7. Système de ventilation respiratoire selon la revendication 6, **caractérisé en ce que**, la première branche d'alimentation en gaz (11) comprend un premier clapet anti-retour inspiratoire (211) et une première vanne de régulation de débit (212) lesquels sont reliés en série, la deuxième branche d'alimentation en gaz (12) comprend un deuxième clapet anti-retour inspiratoire (221) et une deuxième vanne de régulation de débit (222) lesquels sont reliés en série, et la troisième branche d'alimentation en gaz (23) comprend un troisième clapet anti-retour inspiratoire (231) ;
le premier clapet anti-retour inspiratoire (211) est relié à la première interface de source de gaz (11), et le deuxième clapet anti-retour inspiratoire (221) est relié à la seconde interface de source de gaz (12).

8. Système de ventilation respiratoire selon la revendication 7, **caractérisé en ce que**, le dispositif de génération d'oscillation haute fréquence (3) et le troisième clapet anti-retour (231) sont reliés en série ;
la première vanne de régulation de débit (212) et la deuxième vanne de régulation de débit (222) sont respectivement reliées au dispositif de génération d'oscillation haute fréquence (3).

9. Système de ventilation respiratoire selon la revendication 7, **caractérisé en ce que**, le dispositif de génération d'oscillation haute fréquence (3) comprend la première vanne de régulation de débit (212) et la deuxième vanne de régulation de débit (222).

10. Système de ventilation respiratoire selon la revendication 1, **caractérisé en ce que**, le dispositif de commande de ventilation (4) est configuré pour commander, durant la phase inspiratoire, le dispositif expiratoire actif (5) afin de travailler en association avec le dispositif de génération d'oscillation haute fréquence (3) pour extraire une partie du gaz de la branche inspiratoire (2) de sorte à réguler une pression moyenne des voies aériennes.

11. Système de ventilation respiratoire selon la revendication 1, **caractérisé en ce que**, le dispositif de génération d'oscillation haute fréquence (3) est une vanne inspiratoire haute fréquence.

12. Système de ventilation respiratoire selon la revendication 11, **caractérisé en ce que**, la vanne inspiratoire haute fréquence est l'un quelconque d'une électrovanne proportionnelle, d'un robinet de sectionnement, d'une servovanne et d'une turbine.

13. Système de ventilation respiratoire selon la revendication 7, **caractérisé en ce que**, le dispositif expiratoire actif (5) est relié à une sortie du troisième clapet anti-retour inspiratoire (231), ou bien le dispositif expiratoire actif (5) est relié à une branche expiratoire (6).
